# EUROPEAN PATENT APPLICATION

(11) **EP 4 811 374 A1**
(43) Date of publication of application: **23.09.2026**
(21) Application number: 26165455.2
(22) Date of filing: 17.03.2026
(51) Int. Cl.: G16H 20/40

(54) **RADIOTHERAPY GRAPHICAL USER INTERFACES**

(30) Priority: 21.03.2025 US 202519087312
(71) Applicant: Varian Medical Systems, Inc., Palo Alto, CA 94304 (US)
(72) Inventor: SPATOLA, Brian, Palo Alto, 94304 (US); VOJAN, Filip, Palo Alto, 94304 (US)
(74) Representative: Mathisen & Macara LLP

(57) **Abstract**

Embodiments discussed herein provide methods (300) and systems (240) for configuring and guiding radiotherapy treatment workflows using a dynamic user interface integrated into the gantry (224) of a radiotherapy machine (241). In one embodiment, a method (300) includes generating (310) a set of tasks corresponding to different stages of the radiotherapy treatment. The tasks are visually represented (320) on a display screen located on the gantry (224) as a set of radially arranged icons (406-432). Each icon (406-432) corresponds to a specific task and is dynamically updated to reflect task status or provide actionable information. The radial arrangement ensures intuitive access to task-related information, enhances usability during gantry (224) rotation, and maintains icon (406-432) legibility and orientation. This method (300) streamlines treatment setup, facilitates operator interaction, and improves accuracy and efficiency in delivering radiotherapy treatments tailored to individual patients.

## Description

### TECHNICAL FIELD

This application relates generally to graphical user interfaces and software for radiotherapy treatments, radiotherapy setup and/or machines.

### BACKGROUND

Radiation therapy, which utilizes ionizing radiation, is a localized treatment targeting specific tissues, such as cancerous tumors. Ideally, this therapy focuses on the planning target volume (PTV) or target organ, sparing surrounding healthy tissue from excessive radiation doses to minimize damage. To ensure the prescribed dose is accurately delivered to the PTV, the patient must be precisely positioned relative to the linear accelerator, typically using a movable treatment couch on a turntable assembly. Implementing radiation therapy is a complex process involving specific guidelines, protocols, and instructions followed by various medical professionals, including clinicians, technicians, device manufacturers, and treating physicians. Due to the hazards associated with radiation, it is crucial that all instructions are meticulously followed.

Conventional radiation therapy systems are often complex to operate, requiring extensive training for efficient workflow execution. Tasks such as patient positioning, system calibration, and machine adjustments involve numerous interactions with both the patient and the radiotherapy machine. The data needed for these setups is often voluminous and complex, typically displayed on a screen within the treatment room. However, these conventional methods can be inefficient, ineffective, or even dangerous. Conventional radiotherapy systems do not efficiently display the data needed for patient and machine setup. In some conventional systems and methods, the necessary information for an operator (e.g., radiotherapy technician or any other medical professional) is not presented in a manner that is intuitive and user-friendly, interrupting the adjustment process and proving to be ineffective and time-consuming. Some other conventional systems and methods often provide all the information via static and complex graphical user interfaces (GUIs). Unless an operator is highly experienced with a particular system, this can slow down the setup process and degrade the patient's experience.

### SUMMARY

In accordance with a first aspect of the invention, there is provided a computer-readable medium storage as defined by claim 1.

In accordance with a second aspect of the invention, there is provided a computer system as defined by claim 6.

In accordance with a third aspect of the invention, there is provided a method as defined by claim 11.

Optional features are defined by the dependent claims.

Given the complexities involved and discussed herein, there is a need for an intuitive and workflow-oriented graphical user interface (GUI) specific to radiotherapy treatments that efficiently displays treatment setup data, such as machine parameters, patient alignment data, and necessary accessories. Conventional radiotherapy GUIs often lack workflow-oriented design, presenting information and controls in a static or disjointed manner that fails to align with the sequential nature of radiotherapy treatment processes. These interfaces may require operators to navigate through multiple screens or menus to access relevant tasks, increasing cognitive load and the potential for errors during critical procedures. Additionally, conventional GUIs may not provide intuitive visual cues, resulting in inefficiencies as operators struggle to locate and interact with the necessary controls. This lack of alignment with the natural progression of treatment workflows creates challenges in maintaining focus and ensuring precision. There is a need for a more intuitive and user-friendly interface that organizes icons and information in a manner that visually and functionally aligns with the radiotherapy workflow. The methods and systems discussed herein provide an easy-to-use and intuitive display for different radiotherapy icons that correspond to different steps within a radiotherapy workflow. By adopting designs that are logically structured and dynamically responsive, such as radially arranged and interactive icons, operators can easily access and complete tasks, reducing errors, improving efficiency, and enhancing the overall treatment experience.

In one embodiment, the techniques described herein relate to a computer-readable medium storage including a set of non-transitory instructions, that when executed, cause a processor to: generate a set of tasks for configuring a radiotherapy treatment for a patient, at least one task corresponding to at least one stage of the radiotherapy treatment; and present, on a display screen located on a gantry of a radiotherapy machine configured to implement the radiotherapy treatment, a set of icons corresponding to the set of tasks, wherein the set of icons are radially arranged on the display screen.

In some aspects, the techniques described herein relate to a computer-readable medium storage, wherein the set of icons is arranged in accordance with their respective order within the set of tasks.

In some aspects, the techniques described herein relate to a computer-readable medium storage, wherein a position of an icon within the set of icons corresponds to a task category of that icon.

In some aspects, the techniques described herein relate to a computer-readable medium storage, wherein the task category is at least one of a patient setup category, radiotherapy accessory category, radiotherapy machine attribute category, rotational parameter category, interlocks, or a system status category.

In some aspects, the techniques described herein relate to a computer-readable medium storage, wherein a visual attribute of at least one icon corresponds to a task status of its corresponding task.

In some aspects, the techniques described herein relate to a computer-readable medium storage, wherein the set of non-transitory instructions further cause the processor to re-arrange a position of at least one icon responsive to receiving an instruction.

In some aspects, the techniques described herein relate to a computer-readable medium storage, wherein at least one icon indicates a status associated with a room in which the radiotherapy machine is located.

In another embodiment, the techniques described herein relate to a computer system including: a radiotherapy machine; and a processor in communication with the radiotherapy machine, the processor configured to generate a set of tasks for configuring a radiotherapy treatment of a patient, at least one task corresponding to at least one stage of the radiotherapy treatment; and present, on a display screen located on a gantry of the radiotherapy machine configured to implement the radiotherapy treatment, a set of icons corresponding to the set of tasks, wherein the set of icons are radially arranged on the display screen.

In some aspects, the techniques described herein relate to a computer system, wherein the set of icons is arranged in accordance with their respective order within the set of tasks.

In some aspects, the techniques described herein relate to a computer system, wherein a position of an icon within the set of icons corresponds to a task category of that icon.

In some aspects, the techniques described herein relate to a computer system, wherein the task category is at least one of a patient setup category, radiotherapy accessory category, radiotherapy machine attribute category, rotational parameter category, interlocks, or a system status category.

In some aspects, the techniques described herein relate to a computer system, wherein a visual attribute of at least one icon corresponds to a task status of its corresponding task.

In some aspects, the techniques described herein relate to a computer system, wherein the processor is further configured to re-arrange a position of at least one icon responsive to receiving an instruction.

In some aspects, the techniques described herein may relate to a computer system wherein at least one icon indicates a status associated with a room in which the radiotherapy machine is located.

In yet another embodiment, the techniques described herein relate to a method including: generating, by at least one processor, a set of tasks for configuring a radiotherapy treatment for a patient, at least one task corresponding to at least one stage of the radiotherapy treatment; and presenting, by the at least one processor on a display screen located on a gantry of a radiotherapy machine configured to implement the radiotherapy treatment, a set of icons corresponding to the set of tasks, wherein the set of icons are radially arranged on the display screen.

In some aspects, the techniques described herein relate to a method, wherein the set of icons is arranged in accordance with their respective order within the set of tasks.

In some aspects, the techniques described herein relate to a method, wherein a position of an icon within the set of icons corresponds to a task category of that icon.

In some aspects, the techniques described herein relate to a method, wherein the task category is at least one of a patient setup category, radiotherapy accessory category, radiotherapy machine attribute category, rotational parameter category, interlocks, or a system status category.

In some aspects, the techniques described herein relate to a method, wherein a visual attribute of at least one icon corresponds to a task status of its corresponding task.

In some aspects, the techniques described herein relate to a method, further including re-arranging, by the at least one processor, a position of at least one icon responsive to receiving an instruction.

In some aspects, the techniques described herein relate to a method, wherein at least one icon indicates a status associated with a room in which the radiotherapy machine is located.

The methods discussed herein may assist the user in performing a radiotherapy setup workflow, by means of a continued and/or guided process of human-machine interaction. The systems discussed herein may be configured to assist the user in performing a radiotherapy workflow and/or a radiotherapy setup workflow, by means of a continued and/or guided process of human-machine interaction.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a workflow-oriented radiotherapy system, according to an embodiment.
**FIG. 2A** illustrates a radiotherapy machine in a default, upright orientation, according to an embodiment.
**FIG. 2B** illustrates a radiotherapy machine in a rotated orientation, according to an embodiment.
**FIG. 3** is a flow diagram of a method for displaying radiotherapy icons on a radiotherapy machine during rotation of the radiotherapy machine, according to an embodiment.
**FIGS. 4A-4B** illustrate a radiotherapy machine displaying a set of icons, according to an embodiment.
**FIG. 5** illustrate a radiotherapy machine displaying a set of icons, according to an embodiment.

### DETAILED DESCRIPTION

Reference will now be made to some embodiments illustrated in the drawings, and specific language will be used here to describe the same. It will nevertheless be understood that no limitation of the scope of the embodiments of the methods and systems described herein is thereby intended. Alterations and further modifications of the features illustrated here, and additional applications of the principles of the embodiments of the methods and systems described herein as illustrated here, which would occur to a person skilled in the relevant art and having possession of this disclosure, are to be considered within the scope of the embodiments, methods, and/or systems described herein.

### System Architecture

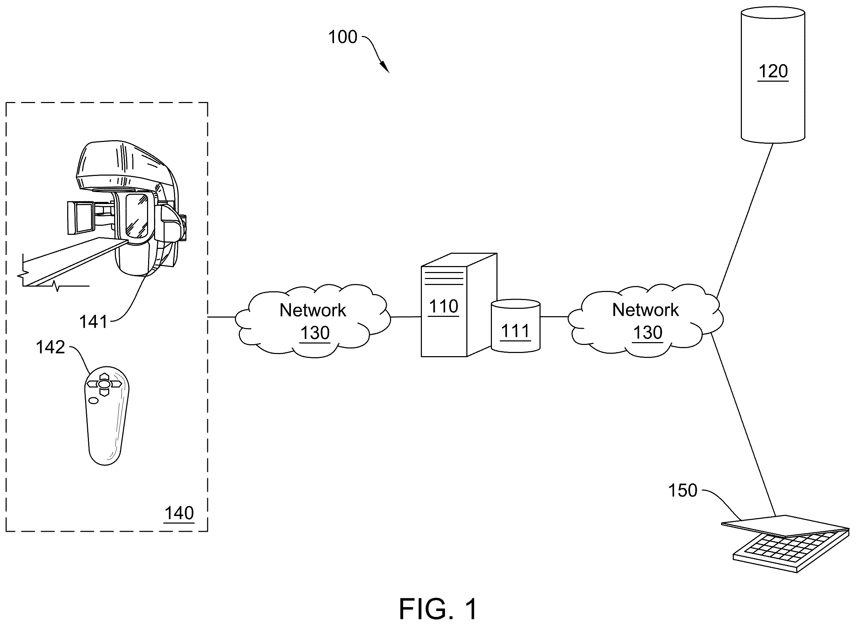

The methods described herein can be implemented using various computing devices/features described in **FIG. 1****.** Therefore, **FIG. 1** describes a non-limiting example of a computer environment where a server can perform the processes/methods described herein, such as retrieving, processing, and presenting radiotherapy treatment data, and presenting data for a graphical user interface (GUI) having workflow-oriented pages (or instances) on various displays screens.

**FIG. 1** illustrates various components of a system **100** for presenting various pages related to operation of a radiotherapy treatment, in accordance with an embodiment. The system **100** may include an analytics server **110,** a medical records database **120,** a radiotherapy system **140,** and a system administrator computer **150** or workstation. These features may communicate with each other over a network **130.** For example, the system **100** may present, optionally via the analytics server **110,** one or more pages/GUIs on the radiotherapy machine **141.** In another example, the system **100** may present, optionally via the analytics server **110,** a page presenting a live feed of a patient on one or more display devices, such as the display device on a gantry of the radiotherapy machine **141.**

The network **130** may include wired and/or wireless communications according to one or more standards via one or more transport mediums. Communication over the network **130** may be in accordance with various communication protocols, such as transmission control protocol and internet protocol (TCP/IP), user datagram protocol (UDP), and Institute of Electrical and Electronics Engineers (IEEE) communication protocols. The network **130** may further include wireless communications according to Bluetooth specification sets, or another standard or proprietary wireless communication protocol. The network **130** may further include communications over a cellular network, including, for example, a global system for mobile (GSM) communications, code division multiple access (CDMA), and enhanced data for global evolution network (EDGE). The examples of the network **130** may include, but are not limited to, private or public local area network (LAN), wireless LAN (WLAN), metropolitan area network (MAN), wide area network (WAN), and the Internet.

The analytics server **110** may be any computing device capable of performing the actions described herein. For instance, the analytics server **110** includes a processing unit and a non-transitory machine-readable storage medium. The processing unit includes a processor with a computer-readable medium, such as a random-access memory coupled to the processor. In some embodiments, the analytics server **110** executes algorithms or computer executable program instructions, which may be executed by a single processor or multiple processors in a distributed configuration. The instructions allow the processor to implement the functionality described herein. The analytics server **110** may be configured to interact with one or more software modules of a same or a different type operating within the system **100.** In general, any of the steps discussed herein as performed by the analytics server may be performed by the system in general.

Non-limiting examples of the processor may include a microprocessor, an application specific integrated circuit, and a field programmable object array, among others. The analytics server **110** may be capable of executing data processing tasks, data analysis tasks, and valuation tasks. Non-limiting examples of the analytics server **110** may include a desktop computer, a server computer, a laptop computer, a tablet computer, and the like. For simplicity, **FIG. 1** depicts a single-server computing device functioning as the analytics server **110.** However, some embodiments may include a plurality of server computing devices capable of performing various tasks described herein.

Implementation of the methods described herein is not limited to the system architecture depicted in **FIG. 1****.** In alternative embodiments, the analytics server **110** may be an embedded computing device disposed within the radiotherapy machine **141.** In some embodiments, the analytics server **110** may be a plurality of computing devices operated locally and/or remotely. In various embodiments, the analytics server **110** may be operated through a cloud service (e.g., network, internet). The cloud service may be performed in accordance with various communication protocols such as TCP/IP, UDP, and IEEE communication protocols. The analytics server **110** may utilize a database, such as a local database **111,** to store and/or retrieve various data described herein. For instance, the analytics server **110** may store different data corresponding to the different pages within the local database **111.** The page may be displayed on a display screen associated with the radiotherapy system **140.**

For instance, the analytics server **110** may display a page having a live feed of the patient before the treatment has begun and/or may display various pages describing how to position the patient on the couch and/or how to adjust the radiotherapy machine **141.** Even though some embodiments describe the analytics server **110** displaying various pages on a display screen attached to the radiotherapy machine **141** (e.g., located on the gantry of the radiotherapy machine **141),** it is expressly understood that the analytics server **110** may display different pages described herein on a display screen located anywhere within the treatment room, such as located on the wall of the treatment room or on any electronic device within the treatment room. In some configurations, the analytics server **110** may display the pages on the system administrator computer **150** or workstation.

The local database **111** may also store data corresponding to the radiotherapy machine **141** (e.g., default position of the radiotherapy machine **141,** and/or current position of the radiotherapy machine **141,** such as the orientation of the bed/couch and/or gantry).

If the analytics server **110** receives a request from the radiotherapy system **140** to provide the current position/orientation of the radiotherapy machine **141,** the analytics server **110** may query the local database **111** and may retrieve the corresponding data. Additionally, or alternatively, the analytics server **110** may also communicate with various sensors associated with the radiotherapy system **140** to retrieve machine data and parameters. The analytics server **110** may then use the methods/systems described herein to instruct the radiotherapy machine **141** to adjust the positioning of the radiotherapy machine **141** to the default position of the radiotherapy machine **141** or another position of the radiotherapy machine, or to display one or more pages instructing a technician to adjust the radiotherapy machine **141** and/or the patient.

The local database **111** may also store data associated with different users of the radiotherapy system **140.** In a non-limiting example, the user data may include the user's login (e.g., sign-in, credentials) information and authorization levels. For example, the analytics server **110** may store, using the local database **111,** the user's name and password. The analytics server **110** may allow the user to log in to the radiotherapy machine **141,** and/or the system administrator computer **150.** If the user logs-in into the system, the analytics server **110** may adjust what the user is able to view, adjust, and control based on the user's authorization level. The analytics server **110** may conceal the patient's medical information that is not relevant to the radiation treatment from the medical technician. In another example, the analytics server **110** may conceal all of the patient's medical information if a machine technician signs-in.

The analytics server **110** may also utilize one or more other databases, such as the medical records database **120,** to store and/or retrieve various data described herein. The databases herein can be configured as one or more databases storing the data, and the disclosure is not intended to be limited to a particular number or location of databases. The analytics server **110** may instruct the medical records database **120** to store patient data (e.g., patient name, patient machine alignment information) associated with a patient identifier (e.g., patient's name, patient's profile image). The analytics server **110** may then instruct the medical records database **120** to populate a dataset corresponding to a patient and display the profile image of the patient. If the analytics server **110** receives a request from the radiotherapy system **140** to display the data associated with the patient identifier, the analytics server **110** may query the medical records database **120** and may retrieve the corresponding dataset. The analytics server **110** may then use the methods/systems described herein to dynamically display the patient data in accordance with the patient identifier.

The medical records database **120** may also include a radiotherapy treatment file associated with the patient identifier. As used herein, the radiotherapy treatment file refers to all data associated with a patient's radiation therapy treatment and is not limited to a particular step or information. The radiotherapy treatment file may also be retrieved from the radiotherapy system **140,** the medical records database **120,** and/or the system administrator computer **150.** The radiotherapy treatment file may include the treatment data associated with a patient. The radiotherapy treatment file may be associated with a patient identifier and may also be updated after a patient has completed treatment. For example, after the patient has completed a treatment session, the medical records database **120** may retrieve the duration of the treatment session from the radiotherapy system **140** and update the radiotherapy file to include the duration of the treatment session. Even though aspects of the embodiments described herein discuss radiotherapy treatment as a file, the radiotherapy treatment file represents a collection of the patient's medical and treatment data, which may be stored in different files. For brevity, the present disclosure refers to the patient's data as a radiotherapy treatment file.

The radiotherapy treatment file may include data for treatment plans for one or more patients where each treatment plan is specific to a single patient. For instance, each treatment plan is uniquely created for each patient and corresponds to the patient's unique attributes (e.g., physical attributes of the patient and the patient's unique condition to be treated). In some configurations, each treatment plan for each patient may itself include multiple files.

The analytics server **110** may retrieve treatment data associated with a patient that is stored within the patient's radiotherapy treatment file(s). As described herein, the analytics server **110** may analyze the treatment data and may display various features and graphical components described herein. While the medical records database **120** may contain treatment data (radiotherapy treatment files) associated with multiple patients, the methods described herein are implemented such that various pages and graphical features described herein are specific to the particular patient being treated.

In some configurations, the analytics server **110** may retrieve radiotherapy treatment files associated with multiple patients. The analytics server **110** may then receive a selection by an operator (e.g., technician) of a patient to be treated. As a result, the analytics server **110** identifies the radiotherapy treatment file associated with the selected patient and customizes the graphical components described herein for the selected patient.

The analytics server **110** may also retrieve and instruct the medical records database **120** to store patient data associated with the patient from the medical records database **120,** the radiotherapy system **140,** and/or the system administrator computer **150.** For instance, the medical records database **120** may include patient data (e.g., previously populated by the analytics server **110** and/or periodically retrieved from a third-party data source). If a patient is selected, the analytics server **110** may query and retrieve patient data from the medical records database **120** and provide the retrieved patient data. For instance, the analytics server **110** may display the patient's profile image when the patient is selected, providing an opportunity to verify the correct patient was selected.

The analytics server **110** may receive treatment data associated with a patient from the medical records database **120,** the treatment data may further include at least a patient identifier. The analytics server may receive radiotherapy treatment file associated with one or more patients from the medical records database **120.** The radiotherapy treatment file may refer to a file having data associated with a process in which a medical team (e.g., radiation oncologists, radiation therapist, medical physicists, and/or medical dosimetrists) plan the appropriate external beam radiotherapy or internal brachytherapy treatment techniques for a patient.

The data within the radiotherapy file is not limited to the external radiation therapy as other treatments may impact the radiation therapy, such as medical oncology treatment (chemotherapy), interventional oncology treatment (e.g. cryotherapy, microwave therapy, or embolic therapy) or other non-treatment procedures, such as labs and appointments with other medical professionals. The radiotherapy treatment file may include data specific to one or more patient's radiotherapy treatment. The radiotherapy treatment file may include any combination of one or more of: a patient identifier, patient's electronic health data records, medical images (e.g., CT scans, 4D CT Scans, MRIs, and x-ray images), treatment-specific data (e.g., arc information or treatment type), target organ (e.g., specification and location data to identify the tumor to be eradicated), treatment plan, etc. Additional examples may include non-target organs, dosage-related calculations (e.g., radiation dose distribution within an anatomical region of the patient), and/or radiotherapy machine specific information (e.g., couch-gantry orientations, machine trajectory, control points, dose distributions, and/or arc information).

The analytics server **110** may use the patient identifier within the radiotherapy treatment file to identify a particular patient and retrieve additional information regarding said patient. For instance, the analytics server **110** may query the medical records database **120** to identify medical data associated with the patient. For instance, the analytics server may query data associated with the patient's anatomy, such as physical data (e.g., height, weight, and/or body mass index) and/or other health-related data (e.g., blood pressure or other data relevant to the patient receiving radiotherapy treatment). The analytics server **110** may also retrieve data associated with current and/or previous medical treatments received by the patient (e.g., prior treatment fractions, or data associated with the patient's previous surgeries).

The analytics server **110** may analyze the data received and generate additional queries accordingly. For instance, the analytics server **110** may retrieve data associated with one or more medical (or other) devices needed for the patient. The analytics server may retrieve data indicating that the patient suffers from a respiratory medical condition. As a result, the analytics server **110** may generate and transmit a query to the radiotherapy machine **141,** or the system administrator computer **150** to identify whether the patient uses/needs a ventilator.

If necessary, the analytics server **110** may also analyze the patient's medical data records to identify the needed patient attributes. For instance, the analytics server **110** may query a database to identify the patient's BMI. However, because many medical records are not digitalized, the analytics server **110** may not receive the patient's BMI value using simple query techniques. As a result, the analytics server **110** may retrieve the patient's electronic health data and may execute one or more analytical protocols (e.g., natural language processing) to identify the patient's body mass index. In another example, if the analytics server **110** does not receive tumor data (e.g., end-points) the analytics server **110** may execute various image recognition protocols and identify the tumor data.

Another example of a patient attribute may include specific tumor locations. More specifically, this data may indicate the primary tumor location with respect to the patient's centerline. This data may be inputted by the treating oncologist or may be analyzed using various image recognition or segmentation methods executed on the patient's medical images.

Another example of a patient attribute may include whether the patient uses prosthesis (e.g., hip or femoral head prosthesis). This attribute may result in a change of the patient's treatment (e.g., patients with these conditions might require a special treatment).

The analytics server **110** may use various application-programming interfaces (APIs) to communicate with different features described herein. As used herein, an API refers to a computing interface that uses connector programming code to act as a software intermediary between at least two computing components/features described herein. The API may automatically and/or periodically transfer various calls, instructions, and/or requests among different features of the system **100.** Using different APIs, the analytics server **110** may automatically transmit and/or receive calls and instructions.

Additionally, or alternatively, the analytics server **110** may use a content delivery network (CDN) to ensure data integrity when communicating with different features described in the system **100.** As described herein, a CDN refers to a distributed delivery network of proxy servers/nodes that uses multi-layered delivery methods/systems to transmit data (e.g., Akamai). The analytics server **110** may use a CDN when communicating various calls/instructions with the network **130** and/or the local database **111.**

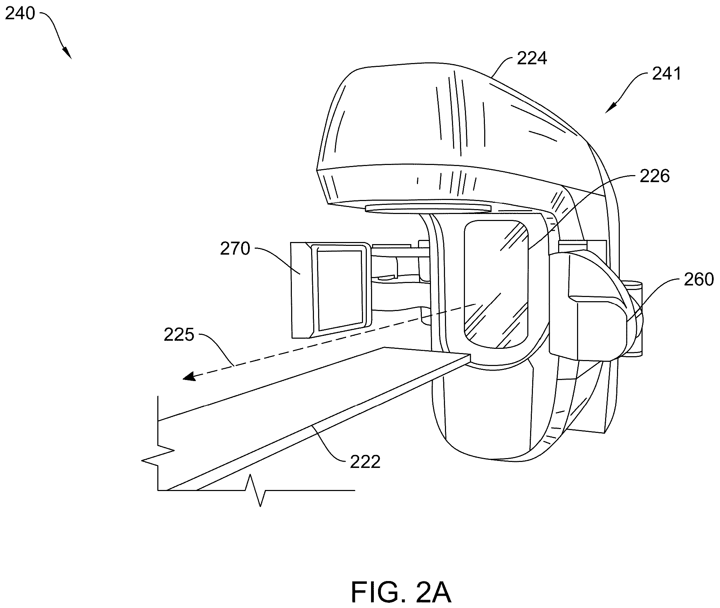

The radiotherapy machine **141** may also include a couch (shown as couch **222** in **FIG. 2A****).** to align the patient in a designated position before the treatment begins. The designated position may be identified, calculated, and/or retrieved by the analytics server **110.** For example, the analytics server **110** may retrieve patient data from the medical records database **120,** analyze the data, and utilize the analyzed data to position the patient on the couch. In some embodiments, the doctor identifies how to align the patient on the couch to optimize the treatment therapy. The radiotherapy machine **141** directs radiation at specified locations of the patient resting on the couch during treatment. The specified locations may be selected by the technician operating the radiotherapy machine **141,** the pendant **142,** and/or the system administrator computer **150.** The analytics server **110** may also specify the locations on the patient to direct the radiation.

The radiotherapy machine **141** may also include an x-ray emitter and an x-ray receiver. The x-ray emitter may be disposed on a gantry of the radiotherapy machine **141** and may be configured to provide x-ray (e.g., a penetrating form of high-energy electromagnetic radiation) waves. The x-ray emitter emits x-ray waves to the patient who is positioned on the couch. The x-ray receiver may be disposed opposed to the x-ray emitter. The x-ray waves received by the x-ray receiver generate an imprint (e.g., image) of the patient's internal anatomy. Although the example embodiment recites the use of x-ray imaging, an alternative configuration for the radiotherapy machine may include an additional or other medical imaging apparatus (e.g., fluoroscopy apparatus, MRI, etc.).

The radiotherapy machine **141** may also include a set of cameras (e.g., camera on an end of the couch, gantry camera, ceiling camera, or other cameras placed within the radiotherapy room). The analytics server **110** may retrieve a live feed of the couch (e.g., with or without the patient) from the set of cameras and provide it to a display screen disposed on the radiotherapy machine **141.** The set of cameras may also directly communicate with the radiotherapy system **140.**

The radiotherapy machine **141** may also include a gantry that may be in communication with the analytics server **110.** If the radiotherapy machine **141** is in operation, the gantry may rotate relative to the radiotherapy machine **141** to begin the treatment of the patient. The analytics server **110** may use the live feed of the set of cameras to control and/or stop the movement of the gantry. During operation, the analytics server **110** may instruct the display screen of the radiotherapy machine **141** to display the live feed of the set of cameras and/or a designated page to the display screen of the radiotherapy machine **141.** In various embodiments, the analytics server **110** may not instruct the display screen of the radiotherapy machine **141** to activate the display screen of the radiotherapy machine **141** during operation. In some embodiments, the display screen of the radiotherapy machine **141** may be a touch screen and may control the pages generated by the analytics server **110** through the display screen of the radiotherapy machine **141.**

The radiotherapy machine **141** may have a default home position. Before the patient receives treatment, the radiotherapy machine **141** may be reset to a position that is ergonomically desirable for the patient. The default home position may also be optimized to receive a new patient or allow the patient to more easily get off the couch.

As discussed in greater detail herein, the radiotherapy machine **141** may also be controlled by the pendant **142.** The pendant **142** may be connected to the radiotherapy machine **141** through wired or wireless communications according to, for example, Bluetooth specification sets or another standard or proprietary wireless communication protocol. In various embodiments, the pendant **142** may be connected to the radiotherapy machine **141** through a wired connection or be integrated into the radiotherapy machine **141.** The pendant **142** may also be in communication with the analytics server **110.** The pendant **142** may adjust the positioning of the radiotherapy machine **141** through a selection of buttons that axially actuates the radiotherapy machine **141** (e.g., couch) towards a desired direction. The technician may also use the pendant **142** to initialize the radiotherapy machine **141,** and/or to have the radiotherapy machine **141** return to its home position.

The pendant **142** may also allow the technician to input patient information, which may be then communicated to the radiotherapy machine **141,** the analytics server **110,** and/or the medical records database **120.** The pendant **142** may be used to control one or more of the steps of the setup and/or treatment of the patient.

The pendant **142** may also include a touch pad (e.g., tactile sensor). The touch pad may be used to control various pages generated by the analytics server **110.** For example, the user may use the touch pad of the pendant **142** to control a page, generated by the analytics server **110,** to proceed through the workflow steps of the radiotherapy setup and/or treatment. In some embodiments, the page may be controlled through a touch screen on the radiotherapy machine **141** or elsewhere in the treatment room.

As discussed in greater detail herein, the radiotherapy system **140** may further include accessories that assist in the radiotherapy setup and/or treatment of the patient. The analytics server **110** may communicate with the radiotherapy system **140** to select which accessories are required. The analytics server **110** may also communicate with the medical records database **120,** and/or the system administrator computer **150** to determine which accessories are required. For example, a patient may require a specific accessory for their radiotherapy setup and/or treatment. The analytics server **110** may retrieve that the patient requires a specific accessory by accessing the medical records database **120** and communicate that information to the radiotherapy system **140.** The accessories may be in communication with the radiotherapy system **140.** For instance, as will be described below, the analytics server may use RFID tags to scan and identify the location and/or presence of various accessories. In some embodiments, the accessories are wired or otherwise integrated into the radiotherapy machine **141.**

The local database **111** associated with the analytics server **110,** the medical records database **120,** and the radiotherapy machine **141** are capable of storing information in various formats and/or encrypted versions. The information may include data records associated with various patient information, user preferences, a set of prompts (e.g., question, query, and inquiry), attributes associated with various pages to be generated by the analytics server **110,** and the like. The medical records database **120,** may have a logical construct of data files, which are stored in non-transitory machine-readable storage media, such as a hard disk or memory, controlled by software modules of a database program (e.g., structured query language (SQL)), and a database management system that executes the code modules (e.g., SQL scripts) for various data queries and management functions.

The system administrator computer **150** may represent a computing device operated by a system administrator. The system administrator computer **150** may communicate with the analytics server **110.** The system administrator computer **150** may be configured to display various analytic metrics where the system administrator can monitor gantry movement, patient information, and modify various thresholds/rules described herein. The system administrator computer **150** may be configurable to display certain analytics metrics when specific thresholds/rules have been exceeded. For example, the system administrator computer **150** may be alerted if a patient has moved a specified amount during treatment. The analytics server **110** may allow the system administrator computer **150** to also control and/or override the settings of the radiotherapy machine **141,** and/or the pendant **142.** For instance, an administrator may revise the minimum distance threshold and/or customize any feature on the pages described herein (e.g., move features within the page, color, font, shape, size, or the order of display for one or more pages).

The analytics server **110** may configure the system administrator computer **150** to review any and/or all commands made through the radiotherapy system **140** at specified process steps. The system administrator computer **150** may then allow or reject the commands made through the radiotherapy system **140.** For example, before the technician starts the patient treatment using the radiotherapy machine **141,** the analytics server **110** may first prompt the system administrator computer **150** to review the radiotherapy machine **141** parameters for approval. Once approved, the technician may then control the radiotherapy machine **141.**

The analytics server **110** may configure the system administrator computer **150** to review any and/or all reading and/or (over)writing of patient data to and/or from the medical records database **120.** For example, before the technician may (over)write patient information collected by the radiotherapy machine **141,** the analytics server **110** may first prompt the system administrator computer **150** to review the patient information before it is stored in the medical records database **120.**

**FIG. 2A** illustrates, as described above, a radiotherapy system **240** including a radiotherapy machine **241** that includes a gantry **224** that rotates about a center axis **225** of the radiotherapy machine **241.** In some embodiments, the radiotherapy system **240** may be substantially similar to the radiotherapy system **140** of **FIG. 1****.** Likewise, the radiotherapy machine **241** may be substantially similar to the radiotherapy machine **141** of **FIG. 1****.** The radiotherapy machine **241** may further include a display screen **226** positioned on or within the gantry **224.** The display screen **226** may be configured to display for view to a user (e.g., a technician, patient, and/or a medical practitioner) relevant information related to the radiotherapy procedure before, during, and/or after the radiotherapy procedure.

The radiotherapy machine **241** may also include an imager **270** and an emitter **260** for administering the radiotherapy to a patient. The radiotherapy system **240** may additionally include a couch **222,** upon which the patient may be placed during the administration of the radiotherapy. In some embodiments, the couch **222** may be translated laterally relative to the radiotherapy machine **241** (e.g., along the center axis **225)** and/or rotated axially relative to the center axis **225** of the radiotherapy machine **241.** Likewise, in some embodiments, the radiotherapy machine **241** may be rotated axially about the center axis **225,** and relative to the couch **222,** before, during, and/or after the administration of the radiotherapy treatment.

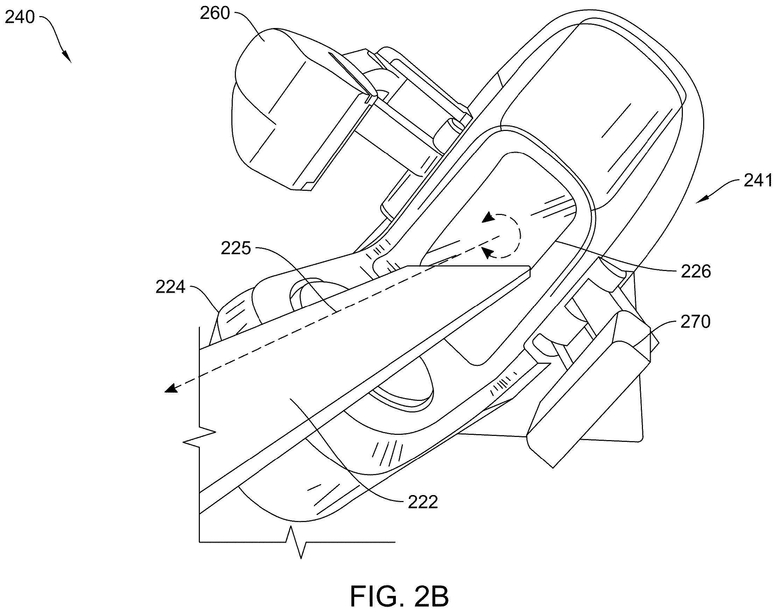

For example, as illustrated in **FIG. 2B****,** the radiotherapy machine **241** is shown in a rotated position about the center axis **225.** Relative to the default position of the radiotherapy machine **241** (as shown in **FIG. 2A****),** the radiotherapy machine **241** in **FIG. 2B** is shown at a position that is rotated about 135.5° counter-clockwise. Because the display screen **226** is coupled to the gantry **224** of the radiotherapy machine **241,** the display screen **226** is also rotated from a default, upright position (as shown in **FIG. 2A****)** at about 135.5° counterclockwise.

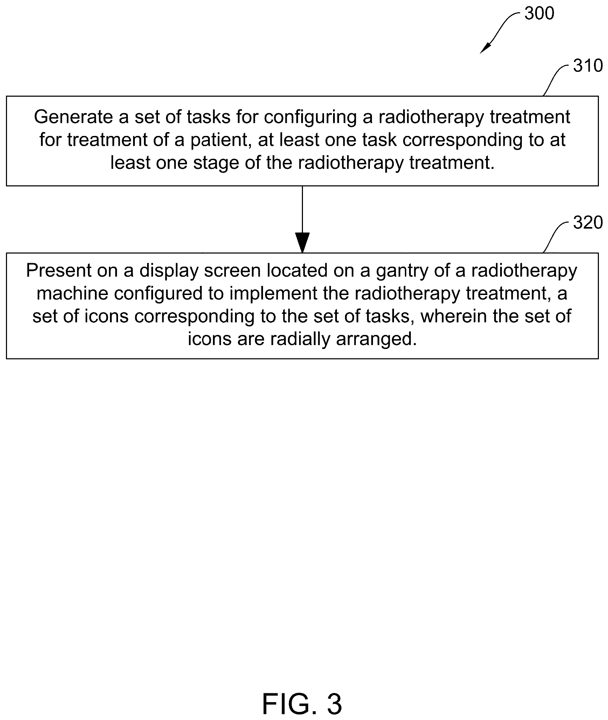

**FIG. 3** is a flowchart of an example method **300** for displaying one or more icons on a display screen coupled to a radiotherapy machine. The method **300** may be executed by one or more processors described herein, such as the analytics server **110,** the pendant **142,** the system administrator computer **150,** the radiotherapy system **140,** the radiotherapy machine **141,** etc. The one or more processors may execute instructions stored on a computer-readable, non-transitory medium, which may cause the one or more processors to execute steps of the method **300.** While the steps of the method **300** are shown in a specific order, it should be understood that the method **300** may comprise more, fewer, and/or different steps than those depicted in **FIG. 3****.** Likewise, the order of the steps of the method **300** is shown in **FIG. 3** for illustrative purposes. However, it is understood that the steps of method **300** may be performed in any number of configurations or orders without departing from the scope of the systems and methods described herein.

At step **310,** one or more processors may generate a set of tasks for configuring a radiotherapy treatment and/or radiotherapy setup of a patient, at least one task corresponding to at least one stage of the radiotherapy treatment and/or radiotherapy setup.

As discussed herein, one or more processors may generate a set of tasks for configuring a radiotherapy treatment and/or radiotherapy setup for a patient. Each task within the set may be associated with a specific stage of the radiotherapy treatment and/or setup, facilitating a structured and workflow-oriented approach to treatment setup, planning and/or execution. In some embodiments, the one or more processors may first retrieve a radiotherapy file (RT file) associated with the patient in order to identify the patient's treatment data.

As used herein, the RT file may refer to a comprehensive collection of data associated with a patient's radiotherapy treatment and/or radiotherapy setup, encompassing all information necessary for setup, planning, executing, and/or monitoring the treatment process. In some embodiments, the RT file may include patient identifiers, such as names and demographic details, along with imaging data like CT scans, 4D CT scans, MRIs, and x-rays that provide anatomical details for treatment planning. The RT file may also contain detailed treatment plans, including beam trajectories, dose levels, arc information, and the type of radiotherapy being administered. Additionally, the RT file may specify target and non-target organ data to ensure accurate radiation targeting while minimizing exposure to healthy tissues.

Additionally, the RT file may include patient-specific attributes such as physical characteristics (e.g., height, weight, BMI) and medical history, as well as details about required accessories like immobilization devices or bolus materials. Session-specific data, such as couch positioning and adjustments from prior treatments, are also recorded to enable consistency across treatment sessions. The RT file may serve as a resource for the one or more processors, enabling the dynamic generation of workflow tasks (e.g., the values displayed for each icon), real-time updates to the GUI, and personalized treatment and/or setup workflows. This structured approach allows the GUI to be populated with precise and efficient radiotherapy and/or radiotherapy setup tailored to the unique needs of each patient. By leveraging the patient's RT file, medical records, and/or treatment data, the one or more processors may identify and define tasks tailored to the patient's unique attributes and treatment requirements.

The set of tasks may correspond to various stages of the radiotherapy treatment and/or radiotherapy setup, such as patient alignment, machine calibration, accessory verification, dose administration, post-treatment analysis, and the like. For instance, during the alignment stage, a task may involve verifying the patient's position on the treatment couch relative to the isocenter of the radiotherapy machine. The one or more processors may generate tasks to guide the operator in adjusting the couch and gantry to achieve the required alignment.

At step **320,** one or more processors may present, on a display screen located on a gantry of a radiotherapy machine configured to implement the radiotherapy treatment and/or radiotherapy setup, a set of icons corresponding to the set of tasks, wherein the set of icons is radially arranged.

In some embodiments, the display screen, integrated into the gantry, may provide a visually accessible/interactive interface that assists operators in efficiently navigating through treatment and/or setup tasks without diverting attention from the patient or the machine. The GUI may display various icons where each icon corresponds to a specific task within the treatment and/or setup workflow, such as patient alignment, machine calibration, or dose administration, and provides intuitive visual indicators to guide the operator through the sequential stages of the treatment and/or setup process.

The set of icons may be radially arranged on the display screen to enhance accessibility and usability during operation. In this way, the icons (or at least a portion of the icons) can be displayed simultaneously. The radial configuration allows operators to easily identify and interact with individual icons regardless of the screen's orientation or the gantry's position. The radial arrangement is designed to optimize spatial organization and ensure that icons remain legible and logically grouped even as the gantry rotates. In some embodiments, the one or more processors may dynamically update the icons based on task completion, visually distinguishing completed tasks and highlighting the next actionable steps in the workflow. This arrangement, combined with real-time updates, improves operational efficiency by reducing cognitive load and enabling seamless progression through the radiotherapy treatment and/or radiotherapy setup process.

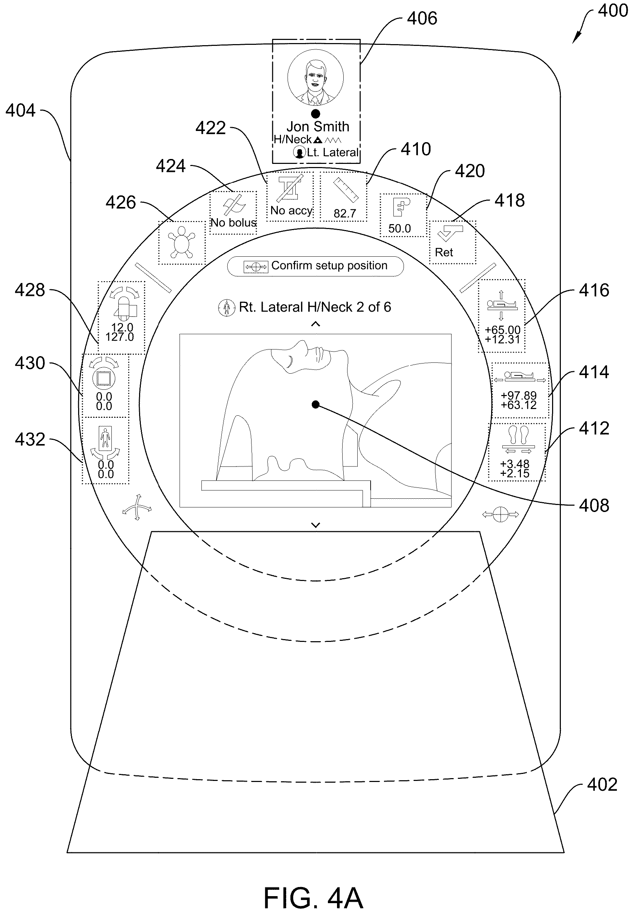

The radial arrangement may place the icons around a center point of the display screen (e.g., center portion **408** discussed in **FIGS. 4A-B**), allowing the icons to be uniformly distributed across the display screen.

In some embodiments, the icons may be displayed radially with each icon positioned at an equal, substantially equal or close to equal, radius from the central point on the display screen. This uniform radial distance may provide a balanced icon layout, where each icon is equidistant or near equidistant from the center point of the display screen, making them easily accessible and visually consistent. Such a configuration minimizes cognitive effort for the operator, as icons are uniformly spaced, and their positions can be intuitively located within the circular arrangement.

The icons may form a full circle or a semi-circle around the central point, depending on the screen size, radiotherapy machine type, the number of icons, and the workflow requirements. In some embodiments, a full circle arrangement may be used for maximizing icon capacity while maintaining symmetry (e.g., when multiple tasks need to be displayed simultaneously). In contrast, a semi-circle arrangement can be used to emphasize a subset of tasks or to make space for additional interface elements, such as dynamic instruction displays or task-specific information. These configurations can adapt dynamically to the operator's needs, presenting icons in a visually organized manner while keeping all relevant information within the same page.

In embodiments where a full-circle arrangement is used, the central point of the display screen could be reserved for detailed task instructions, with the icons evenly distributed around it. In a semi-circle arrangement, the bottom half of the screen could be left open for visual indicators, such as task progress bars or alerts, while the upper semi-circle (and sometimes side portions) may contain the workflow icons. The radial design, whether a full or partial circle, ensures that the icons remain logically grouped and easy to access, even as the gantry or display rotates, maintaining operator efficiency and usability.

The layout of the radial display may allow the operator to easily locate and interact with each icon regardless of the gantry's rotation or the display's orientation. By organizing the icons radially, the system minimizes visual clutter while maintaining a logical grouping of the icons.

The icons displayed on the display screen may be grouped based on predefined attributes such as function, workflow stage, or type of information, and these groupings can be customized or modified to suit the preferences of different operators or clinics. For instance, icons related to patient information, such as demographic data, setup and/or treatment specifics, may be grouped separately from icons representing machine setup tasks, such as couch alignment or gantry positioning. The predefined grouping criteria may allow for consistency across workflows while allowing for flexibility to adapt to varying clinical protocols or individual operator needs. Customization options enable clinics to tailor the grouping and presentation of icons based on their unique workflows, equipment configurations, or operational preferences. This adaptability can be used to ensure that the system remains intuitive and efficient, regardless of the user's specific requirements or the treatment environment.

In some embodiments, the icons may be arranged in a sequential order that reflects the typical (e.g., pre-defined) workflow of the treatment and/or setup process, ensuring tasks are performed in the correct sequence and guiding the operator intuitively through each stage. As a result, the location of the icons may correspond to the order of the tasks. For instance, the icons may be arranged such that a subsequently displayed icon (when considering the display of the icons clockwise or counterclockwise depending on the setup of the icons) may correspond to the next task to be accomplished.

In a non-limiting example, the icon arrangement starts with a patient identification icon, placed at the beginning (most left or most right of the displayed icons) to allow the operator to verify the patient's identity and treatment plan, ensuring the correct individual is being treated. This foundational step minimizes the risk of errors and sets the stage for subsequent tasks. Following this, all other icons may be arranged in a clockwise manner, such that the operator can intuitively understand which task must be performed next.

This logical and workflow-oriented arrangement of icons ensures that operators are intuitively guided through the radiotherapy and/or radiotherapy setup process, reducing cognitive load and minimizing errors. The sequence of icons can also be dynamically adjusted by the analytics server based on real-time task completion or user inputs, providing flexibility to accommodate specific scenarios or operator preferences. This approach ensures that the GUI remains efficient, user-friendly, and aligned with the needs of the radiotherapy and/or radiotherapy setup workflow.

In addition to corresponding to a specific task, each icon may also be dynamically interactive, adapting to changes in the treatment environment and user inputs. This dynamic adaptation may be driven by the analytics server (or any other server/processors discussed herein), which monitors the progress of each task and transitions the GUI to display/highlight the next set of icons upon task completion. For instance, once the alignment of the patient is verified, the corresponding icon may be updated (e.g., visually by changing colors) to indicate completion, while the next icon highlights the verification of dose calibration. This sequential and responsive design ensures that the operator is guided seamlessly through the treatment and/or setup workflow without needing to be directed to a new page and while the viewing status of other icons/tasks, enhancing efficiency and reducing setup time. Moreover, the distinct visual and functional characteristics of each icon-such as color changes, animations, or text overlays-serve as intuitive indicators of task status, enabling quick recognition and action by the operator.

Referring now to **FIG. 4A****,** a display of the icons on a display screen of a radiotherapy machine is presented, in accordance with one embodiment. The set of icons, in this embodiment **400,** are presented on a display screen **404** (similar to the display screen **226)** located on a gantry of a radiotherapy machine having a couch **402** (similar to the couch **222).** As depicted, the icons **406-432** are displayed radially on the display screen **404,** though in other embodiments, the arrangement may be different. The display screen may also include a center portion **408** that can display additional data needed or otherwise associated with different icons.

As depicted, the set of displayed icons includes an icon **406** that may include the patient's personally identifiable information, such as the patient's image, name, diagnosis, and other information that can identify the patient.

Different groupings of icons (or sometimes individual icons) may be arranged in accordance with a predefined order that may or may not correspond to the workflow of the patient's radiotherapy treatment and/or radiotherapy setup. For instance, in one embodiment, different tasks to be performed may be associated with a defined order. As a result, their corresponding icons may be arranged in that order. That is, a position of an icon may correspond to its relative position within a defined order of tasks.

In other embodiments, such as the depicted embodiment, the icons may be arranged in accordance with a grouping of the tasks. For instance, icons may be arranged based on whether they are associated with a patient setup category, radiotherapy accessory category, radiotherapy machine attribute category, rotational parameter category, interlocks, or a system status category. As depicted, the set of icons may be divided into three groups where the groups indicate a cluster of tasks to be performed. The cluster of tasks may have a common attribute and may be clustered based on the common attribute. For instance, one grouping or cluster of icons may indicate adjustments to be made to the patient (e.g., patient alignment) and another grouping of the icons may be directed to machine adjustment while another grouping of icons may be directed to the treatment room or machine attributes (e.g., whether a door of the treatment room is open or whether the pendant of the radiotherapy machine is properly tethered to or otherwise connected to the system).

In the depicted embodiment, a first grouping of the icons may include patient alignment indicators (e.g., icons **412, 414,** and **416).** The icon **412** may indicate whether the patient needs to be laterally moved (e.g., moved to the left or right). Additionally or alternatively, the set of icons may include an icon **414** that indicates whether the patient should be moved on the couch (e.g., toward the radiotherapy machine or away from the machine). Additionally or alternatively, the set of icons may include an icon **416** that indicates whether the patient on the couch should be moved vertically (e.g., whether the couch should be moved up or down).

A second grouping of icons may include machine/treatment accessories and may include icons **410, 418, 420, 422,** and **424.** The icon **410** may correspond to a distance indicator measuring/indicating the distance between the radiation source and the patient's skin (source-to-skin distance, SSD). Additionally or alternatively, the set of icons may include an icon **418** that indicates whether an imager of the machine is extended. Additionally or alternatively, the set of icons may include an icon **420** that indicates a status of the imagers of the machine (e.g., how far the images are from the machine). This information may be needed to ensure that the patient does not collide with the imager during the treatment and/or treatment setup. Additionally or alternatively, the set of icons may include an icon **422** that indicates whether an accessory (and if so, which accessory) is needed to treat the patient. Additionally or alternatively, the set of icons may include an icon **424** that indicates whether a bolus is needed to treat the patient. Additionally or alternatively, the set of icons may include an icon **426** that indicates the patient orientation (e.g., whether the patient should be on their back with their head toward the machine or otherwise). Additionally or alternatively, the set of icons may include an icon **428** that indicates whether any adjustment to the gantry is needed. The icon **428** may include arrows indicating which direction the gantry should be turned or otherwise moved towards. In some embodiments, the icon **428** may also include an angle value indicating a desired position of the gantry.

Another grouping of the icons may include machine attribute icons that correspond to tasks related to adjusting the radiotherapy machine. This group may include icons **428, 430,** and **432.** The icon **430** may indicate whether any adjustments are needed to be made to the collimator. In some embodiments, the icon **430** may include a map of the collimator opening. Additionally or alternatively, the set of icons may include an icon **432** indicating a couch rotation angle (yaw).

In some embodiments, system administrators or operators may have the capability to adjust any attribute of one or more of the icons displayed on the display screen, including visual attributes such as color, font style, size, line thickness, shading, and the like. This flexibility allows the icons to be modified or rearranged to align with institutional branding, operator preferences, or accessibility requirements. For instance, line thickness or font size can be increased to enhance visibility for users with visual impairments. These adjustments ensure that the interface remains both functional and user-friendly, tailored to meet the specific needs of the clinical setting.

The position of the icons on the display screen can also be adjusted by system administrators or operators to optimize usability and align with operator workflows. Icons can be repositioned within the radial arrangement or moved to alternative layouts, ensuring that the most frequently accessed icons are conveniently located. This customization enables the interface to adapt to specific clinic requirements or individual user preferences, improving efficiency during radiotherapy and/or radiotherapy setup procedures. For instance, a clinic focusing on particular types of treatments and/or setup may prioritize the placement of certain icons for quick access, minimizing navigation time and enhancing operational precision.

In some embodiments, a system administrator or an operator can be authorized to add new icons to better reflect the workflow or accommodate updates to the radiotherapy and/or setup process. This capability ensures that the interface remains relevant and streamlined, reducing visual clutter and focusing only on the tasks and information pertinent to the specific clinical scenario. For example, an icon can be added to the set of icons that indicates any combination of one or more of: whether a door of the treatment room is open, whether a predicted collision is going to occur (e.g., dry run), whether the pendant is properly connected to the radiotherapy machine, and the like.

The icons displayed may be interactive, such that the operator can move along the icons and click (or otherwise interact with different icons), e.g., using a pendant of the radiotherapy machine. As icons are selected, the center portion **408** of the display may be dynamically populated with detailed information and/or actionable instructions necessary to accomplish the tasks associated with the selected icons. This dynamic content updating provides the operator with relevant and context-specific data without overwhelming the interface with unnecessary information. By tailoring the displayed content to the specific task at hand, the system enhances operational efficiency and reduces the risk of error by offering clear, task-focused guidance in real-time.

For example, selecting an icon related to patient alignment could populate center portion **408** with a detailed visualization of the patient's current position relative to the isocenter of the machine. It may display numerical values for vertical, lateral, and longitudinal shifts required to achieve proper alignment, alongside graphical indicators showing the misalignment and required corrections. This allows the operator to make precise adjustments to the couch or gantry, ensuring the patient is positioned correctly for the delivery of the prescribed dose, all while keeping the process intuitive and visually guided.

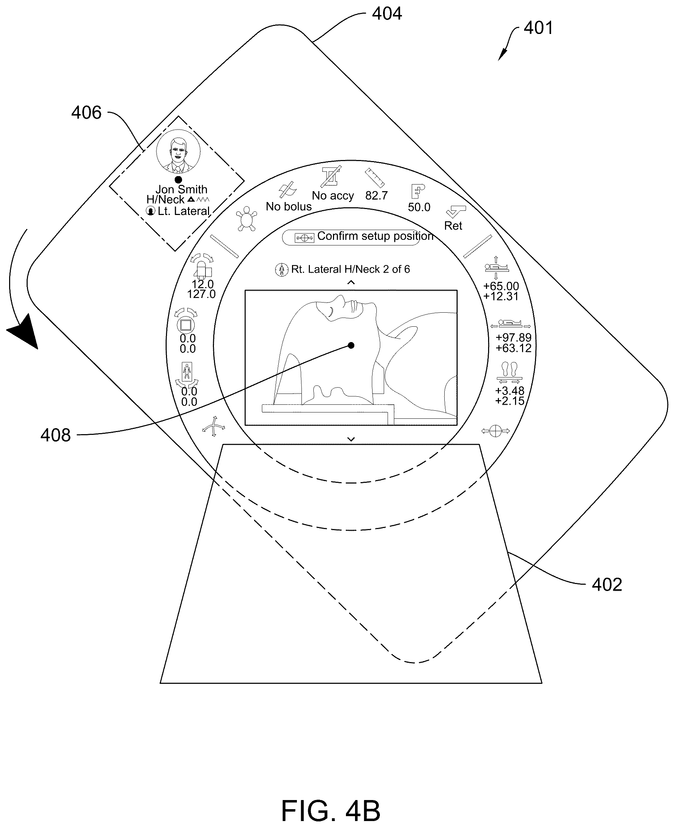

The GUI displayed on the display screen **404,** as shown in **FIG. 4B****,** demonstrates an embodiment **401** where the icons on the display screen **404** are dynamically adjusted to appear stationary to the operator, even as the gantry rotates, causing the display screen **404** itself to rotate. This functionality is achieved by calculating the movement of the gantry and correspondingly relocating and reorienting the icons on the display. In this manner, the visual position and orientation of the icons remain consistent relative to the operator's viewpoint and the room's reference frame, such as the treatment couch or gravity.

For example, as the gantry rotates counterclockwise, as depicted in **FIG. 4B****,** the display screen **404** also rotates along with it. However, the icons and critical information, such as those shown in center portion **408,** are dynamically repositioned and reoriented by the system to maintain their upright orientation and stationary appearance. This ensures that the operator can seamlessly interact with the interface without being affected by the physical movement of the gantry. Such a design significantly enhances usability, particularly during complex workflows, by maintaining the legibility and accessibility of the icons regardless of the gantry's position. In another example, the icon **406** may appear to be rotating with the gantry, however, it is reoriented, such that it is still legible and seems "straight" to the operator. Optionally, any combination of one or more of the icons may be reoriented as the gantry rotates, such that the reoriented icons appear upright as the gantry rotates.

In some embodiments, the visual attribute(s) of at least one icon may correspond to the task status of its associated task. These visual attributes may include, but are not limited to, color, opacity, blinking, font style, size, or line thickness. The one or more processors may dynamically update these attributes based on real-time changes in the task's progress or status. For instance, an icon may be displayed in a specific color (e.g., orange) when a task is outstanding, change to another color when in progress, and adopt yet another visual style or color upon task completion. This allows operators to quickly assess the state of various tasks at a glance, enhancing workflow efficiency and reducing the likelihood of errors.

In some embodiments, the one or more processors may rearrange the position of at least one icon displayed in response to receiving an instruction. This rearrangement allows the interface to adapt dynamically to user preferences, workflow requirements, or real-time operational adjustments. Instructions for repositioning an icon may be provided by an operator, a system administrator, or an automated system component, such as an analytics server. The updated position of the icon ensures that essential information or controls are conveniently located for the operator, optimizing usability and reducing navigation time during critical procedures. In a non-limiting example, different icons may be moved to a different location of the display screen as their corresponding tasks are completed.

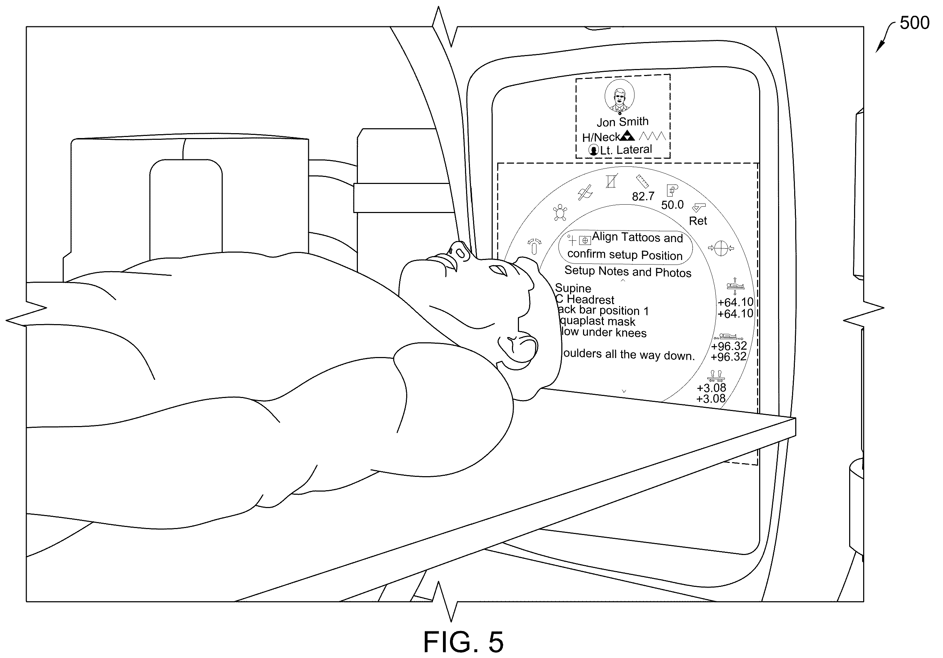

**FIG. 5** illustrates a radiotherapy treatment setup **500,** illustrating a patient positioned on the treatment couch of the radiotherapy machine. The display screen, as depicted, may be integrated into the gantry and visible in the background, is dynamically presenting task-specific information and guidance to the operator. The interface is configured with a radial arrangement of icons, which remain oriented and legible despite any rotation of the gantry. This ensures that the operator can seamlessly interact with the interface regardless of the machine's orientation.

In this setup, the central portion of the display dynamically updates to provide detailed information relevant to the current stage of the radiotherapy workflow. For instance, it may display notes on patient alignment, anatomical landmarks, or the positioning instructions needed to ensure accurate delivery of the prescribed radiation dose and/or to ensure effective radiotherapy setup. The patient's profile and treatment status are also prominently displayed at the top of the interface for easy reference.

### Example clauses

Further aspects of these teachings are provided by the subject matter of the following clauses.

Clause 1. A computer-readable medium storage comprising a set of non-transitory instructions, that when executed, cause at least one processor to: generate a set of tasks for configuring a radiotherapy treatment for a patient, at least one task corresponding to at least one stage of the radiotherapy treatment and/or setup; and present, on a display screen located on a gantry of a radiotherapy machine configured to implement the radiotherapy treatment, a set of icons corresponding to the set of tasks, wherein the set of icons are radially arranged on the display screen.

Clause 2. The computer-readable medium storage of clause 1, wherein the set of icons is arranged in accordance with their respective order within the set of tasks.

Clause 3. The computer-readable medium storage of clauses 1-2, wherein a position of an icon within the set of icons corresponds to a task category of that icon.

Clause 4. The computer-readable medium storage of clauses 1-3, wherein the task category is at least one of a patient setup category, radiotherapy accessory category, radiotherapy machine attribute category, rotational parameter category, interlocks, or a system status category.

Clause 5. The computer-readable medium storage of clauses 1-4, wherein a visual attribute of at least one icon corresponds to a task status of its corresponding task.

Clause 6. The computer-readable medium storage of clauses 1-5, wherein the set of non-transitory instructions further cause the processor to re-arrange a position of at least one icon responsive to receiving an instruction.

Clause 7. The computer-readable medium storage of clauses 1-6, wherein at least one icon indicates a status associated with a room in which the radiotherapy machine is located.

Clause 8. A computer system comprising: a radiotherapy machine; and at least one processor in communication with the radiotherapy machine, the at least one processor configured to generate a set of tasks for configuring a radiotherapy treatment of a patient, at least one task corresponding to at least one stage of the radiotherapy treatment and/or setup; and present, on a display screen located on a gantry of the radiotherapy machine configured to implement the radiotherapy treatment, a set of icons corresponding to the set of tasks, wherein the set of icons are radially arranged on the display screen.

Clause 9. The computer system of clause 8, wherein the set of icons is arranged in accordance with their respective order within the set of tasks.

Clause 10. The computer system of clauses 8-9, wherein a position of an icon within the set of icons corresponds to a task category of that icon.

Clause 11. The computer system of clauses 8-10, wherein the task category is at least one of a patient setup category, radiotherapy accessory category, radiotherapy machine attribute category, rotational parameter category, interlocks, or a system status category.

Clause 12. The computer system of clauses 8-11, wherein a visual attribute of at least one icon corresponds to a task status of its corresponding task.

Clause 13. The computer system of clauses 8-12, wherein the at least one processor is further configured to re-arrange a position of at least one icon responsive to receiving an instruction.

Clause 14. A method comprising: generating, by at least one processor, a set of tasks for configuring a radiotherapy treatment for a patient, at least one task corresponding to at least one stage of the radiotherapy treatment and/or setup; and presenting, by the at least one processor on a display screen located on a gantry of a radiotherapy machine configured to implement the radiotherapy treatment, a set of icons corresponding to the set of tasks, wherein the set of icons are radially arranged on the display screen.

Clause 15. The method of clause 14, wherein the set of icons is arranged in accordance with their respective order within the set of tasks.

Clause 16. The method of clauses 14-15, wherein a position of an icon within the set of icons corresponds to a task category of that icon.

Clause 17. The method of clauses 14-16, wherein the task category is at least one of a patient setup category, radiotherapy accessory category, radiotherapy machine attribute category, rotational parameter category, interlocks, or a system status category.

Clause 18. The method of clauses 14-17, wherein a visual attribute of at least one icon corresponds to a task status of its corresponding task.

Clause 19. The method of clauses 14-18, further comprising: re-arranging, by the at least one processor, a position of at least one icon responsive to receiving an instruction.

Clause 20. The method of clauses 14-19, wherein at least one icon indicates a status associated with a room in which the radiotherapy machine is located.

Clause 21: A computer program comprising instructions which, when the program is executed by a computer, cause the computer to carry out the method of any of clauses 14-20.

Clause 22: A computer-readable medium comprising a set of instructions, that when executed by one or more processors, cause the one or more processors to carry out the method of any of clauses 14-20.

The presentation of information on the display screen, the user interaction with the display screen and/or the GUI, may have the technical effect of assisting the user in performing a radiotherapy setup workflow by means of a continued and/or guided process of human-machine interaction. The systems discussed herein may be configured to assist the user in performing a radiotherapy workflow and/or a radiotherapy setup workflow, by means of a continued and/or guided process of human-machine interaction.

In this disclosure, the term "radiotherapy treatment" may refer to radiotherapy treatment and/or radiotherapy treatment setup. The method steps may refer to treatment setup, while the systems may be configured to perform treatment and/or treatment setup.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the invention. Thus, the operation and behavior of the systems and methods were described without reference to the specific software code being understood that software and control hardware can be designed to implement the systems and methods based on the description herein.

When implemented in software, the functions may be stored therein as one or more instructions or code on a computer-readable, non-transitory medium or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the present invention. Various modifications to these embodiments will be readily apparent to those skilled in the art, and the generic principles defined herein may be applied to other embodiments without departing from the scope of the invention. Thus, the present invention is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A computer-readable medium storage comprising a set of non-transitory instructions, that when executed, cause at least one processor to:
generate a set of tasks for configuring a radiotherapy treatment for a patient, at least one task corresponding to at least one stage of the radiotherapy treatment; and
present, on a display screen coupled to a radiotherapy machine configured to implement the radiotherapy treatment, a set of icons corresponding to the set of tasks,
wherein the set of icons are radially arranged on the display screen.

2. The computer-readable medium storage of claim 1, wherein the set of icons is arranged in accordance with their respective order within the set of tasks.

3. The computer-readable medium storage of claim 1 or claim 2, wherein a position of an icon within the set of icons corresponds to a task category of that icon;
and optionally:
wherein the task category is at least one of a patient setup category, radiotherapy accessory category, radiotherapy machine attribute category, rotational parameter category, interlocks, or a system status category.

4. The computer-readable medium storage of any preceding claim, wherein a visual attribute of at least one icon corresponds to a task status of its corresponding task.

5. The computer-readable medium storage of any preceding claim, wherein the set of non-transitory instructions further cause the at least one processor to re-arrange a position of at least one icon responsive to receiving an instruction;
and/or:
wherein at least one icon indicates a status associated with a room in which the radiotherapy machine is located.

6. A computer system comprising:
a radiotherapy machine; and
at least one processor in communication with the radiotherapy machine, the at least one processor configured to
generate a set of tasks for configuring a radiotherapy treatment of a patient, at least one task corresponding to at least one stage of the radiotherapy treatment; and
present, on a display screen coupled to the radiotherapy machine configured to implement the radiotherapy treatment, a set of icons corresponding to the set of tasks,
wherein the set of icons are radially arranged on the display screen.

7. The computer system of claim 6, wherein the set of icons is arranged in accordance with their respective order within the set of tasks.

8. The computer system of claim 6 or claim 7, wherein a position of an icon within the set of icons corresponds to a task category of that icon;
and optionally:
wherein the task category is at least one of a patient setup category, radiotherapy accessory category, radiotherapy machine attribute category, rotational parameter category, interlocks, or a system status category.

9. The computer system of any of claim 6 to claim 8, wherein a visual attribute of at least one icon corresponds to a task status of its corresponding task.

10. The computer system of any of claim 6 to claim 9, wherein the at least one processor is further configured to re-arrange a position of at least one icon responsive to receiving an instruction.

11. A method comprising:
generating, by at least one processor, a set of tasks for configuring a radiotherapy treatment for a patient, at least one task corresponding to at least one stage of the radiotherapy treatment; and
presenting, by the at least one processor on a display screen coupled to a radiotherapy machine configured to implement the radiotherapy treatment, a set of icons corresponding to the set of tasks,
wherein the set of icons are radially arranged on the display screen.

12. The method of claim 11, wherein the set of icons is arranged in accordance with their respective order within the set of tasks.

13. The method of claim 11 or claim 12, wherein a position of an icon within the set of icons corresponds to a task category of that icon;
and optionally:
wherein the task category is at least one of a patient setup category, radiotherapy accessory category, radiotherapy machine attribute category, rotational parameter category, interlocks, or a system status category.

14. The method of any of claim 11 to claim 13, wherein a visual attribute of at least one icon corresponds to a task status of its corresponding task.

15. The method of any of claim 11 to claim 14, further comprising:
re-arranging, by the at least one processor, a position of at least one icon responsive to receiving an instruction;
and/or:
wherein at least one icon indicates a status associated with a room in which the radiotherapy machine is located.
